# EUROPEAN PATENT APPLICATION

(11) **EP 3 045 185 A1**
(43) Date of publication of application: **20.07.2016**
(21) Application number: 15151366.0
(22) Date of filing: 16.01.2015
(51) Int. Cl.: A61M 5/142, A61M 5/148, A61M 5/162, A61M 5/24, A61M 39/00

(54) **CONNECTOR FOR A CONTAINER FILLED WITH A LIQUID MEDICAMENT**

(71) Applicant: Sanofi-Aventis Deutschland GmbH, 65929 Frankfurt am Main (DE)
(72) Inventor: Teucher, Axel, 65926 Frankfurt am Main (DE); Jugl, Michael, 65926 Frankfurt am Main (DE); Blancke, Stefan, 65926 Frankfurt am Main (DE)
(74) Representative: Weilnau, Carsten

(57) **Abstract**

The present invention relates to a connector for a container (10) filled with a liquid medicament (13) and having a wall structure (12) with at least one flexible portion (18), the connector comprising:
- a body (22) forming a suction chamber (23) to engage with the flexible portion (18),
- a piercing element (28) extending through the body (22) and into the suction chamber (23),
- a suction outlet (30) in fluid communication with the suction chamber (23).

## Description

### Technical field

The present invention relates to the field of flexible containers for injection devices comprising a flexible bag filled with a liquid medicament to be injected or delivered by the injection device.

### Background

Drug delivery devices for administering liquid medicaments are widely known in the art. Parenteral administering of liquid medicaments is typically conducted by means of injection devices, such like syringes, pen-type injectors or by means of infusion pumps, e.g. by way of micropumps.

For treatment of chronic diseases, such like diabetes the medicament has to be regularly administered according to a predefined schedule. Known drug delivery devices may either be adapted for discrete use for injecting of a predefined amount of the medicament a given number of times during the day. Alternatively, such drug delivery devices may be adapted for continuous or quasi-continuous delivery of the medicament through a permanent fluid connection between the delivery device and the patient. Continuous or constant administering of the medicament is typically conducted by means of infusion pumps that are relatively expensive.

Such drug delivery devices typically comprise a container or reservoir to accommodate the liquid medicament and having an outlet in fluid communication with some kind of infusion or injection needle. Moreover, such drug delivery devices also comprise a drive mechanism that is operable to expel or to withdraw a predefined amount of the liquid medicament from the container or reservoir and through the infusion or injection needle into biological tissue of the patient.

There exist reusable as well as disposable devices, wherein with reusable devices the medicament-containing reservoir or container is to be replaced when empty. With disposable drug delivery devices a pre-filled reservoir is non-detachably arranged in the device. When the medicament contained therein has been used up the entire device is intended to be discarded.

Traditionally, vitreous or glass cartridges have been widely used in injection or infusion systems to contain or to accommodate the liquid medicament, hence a particular pharmaceutical composition. Glass cartridges, vials or carpules provide a large degree of optical transparency and are substantially inert to the medicament. This means, that substantially no interaction between the medicament and the glass cartridge takes place even under long term storage conditions, i.e. when the medicament is stored and contained in the cartridge for time intervals of severely years.

Vitreous cartridges or glass cartridges are prone to mechanical impact and may therefore represent a concern for patients but as well for the pharmaceutical industry. Glass breakage typically represents a hazard for the patient as well as for the industrial production environment. Moreover, handling of broken glass is quite risky and dangerous for the persons concerned with a broken cartridge.

Especially with highly concentrated medicaments and with infusion pump applications comparatively small volumes have to be injected or low volume flow rates have to be realized. Extraction and withdrawal of a comparatively small amount of medicament from a vitreous cartridge may be rather elaborate since a piston typically sealing a proximal end of the cartridge is to be displaced in distal, hence in injection direction typically by means of a plunger of the drug delivery device. For such application scenarios use of a deformable or flexible container or reservoir would be advantageous. As the medicament is sucked or withdrawn from the interior of the container the container is subject to a modification of its geometric shape and may start to collapse.

Containers filled with a liquid medicament are typically pierced or punctured by a cannula or a similar piercing element by way of which the liquid content of the container can be withdrawn therefrom. With many injection devices or drug delivery devices access to the interior of a flexible container is obtained by means of a piercing assembly, wherein a piercing element, such like a cannula or injection needle is displaceable relative to the injection device and relative to the flexible container in order to pierce a sidewall or a seal thereof.

Known piercing assemblies typically constitute a number of movable components by way of which a longitudinal or axial displacement of a piercing element can be realized. If the flexible container is to replaceably or detachably arranged in or at an injection device the piercing assembly must also provide a respective retraction of the piercing element in order to liberate the flexible container and to enable a replacement thereof. A piercing mechanism implemented in conventional piercing assemblies to interact with flexible containers therefore requires the implementation of numerous moving parts and eventually a rather delicate adjustment of their mutual mechanical interaction.

### Objects of the Invention

It is therefore an object of the present invention to provide an improved connector for a container filled with a liquid medicament, which container has a wall structure with at least one flexible portion. The connector should be implementable with only a limited number of components. It is a further aim that the connector does not require and is hence void of any movable parts. It is a further aim, that the connector is rather robust, reliable and failure-safe. The connector should be particularly suitable to establish repeated fluid-transferring connection and respective disconnections to and from the interior volume of a container or medicament reservoir.

### Summary

In a first aspect the invention relates to a connector for a container filled with a liquid medicament. In particular, the connector is configured for and adapted to connect to a flexible container, e.g. having a flexible bag. However, the connector is not limited to containers being entirely flexible. It is only required that the container comprises a wall structure with at least one flexible portion.

The connector comprises a body forming a suction chamber to engage with the flexible portion of the container's wall structure. The connector further comprises a piercing element extending through the body and into the suction chamber. In addition the connector comprises a suction outlet in fluid communication with the suction chamber. Here, the suction outlet distinguishes from the piercing element. While the piercing element is intended to pierce the flexible portion of the container to gain access to the interior of the container the suction outlet is operable to decrease the pressure inside the suction chamber. In this way, the suction outlet is particularly operable to reduce and to lower the pressure inside the suction chamber to a level that is substantially smaller than a pressure level inside the container. As a consequence and by establishing a low pressure of a desired level in the suction chamber the flexible portion of the container's wall structure will be dragged or sucked into the suction chamber at least to such a degree that the flexible portion is pierced and penetrated by the piercing element. The piercing element may be arranged and fixed in an immobile way to the body of the connector. In effect the connector is void of any moving or movable parts.

By means of the body forming the suction chamber, and further by means of the piercing element and the suction outlet a fluid communicating connection between piercing element and the interior of the container can be established exclusively by a low pressure-induced local deformation of the wall structure of the container. In this way, the connector does not require any movable components to generate a liquid transferring connection between the piercing element and the container's interior. In the same way as the flexible portion of the wall structure is suckable into the suction chamber it may also relax due to elastic restoring forces as soon as the pressure inside the suction chamber raises to a level in a region of a pressure level inside the container.

The connector therefore provides a detachable and releasable mutual connection of the piercing element with the interior of the container without a single moving part but only on the basis of a pressure control and/or pressure regulation inside the suction chamber.

According to an embodiment the suction chamber is formed by a bottom and by a sidewall connected thereto. Typically, the bottom is connected with the piercing element. The piercing element typically configured as a hollow and tipped cannula or injection needle is fixed to the bottom and extends through the bottom. A proximal and tipped end of the piercing element is located inside the suction chamber formed by the bottom and the sidewall whereas a distal end of the piercing element is located outside the suction chamber. It may protrude from the bottom thereof. The distal end of the piercing element may be connected to a tube or a similar fluid guiding structure.

The sidewall is connected to the bottom and extends at a predefined angle from the bottom. Typically, the bottom is of substantially planar shape and the piercing element is located in the center of the bottom. The sidewall extends around the outer circumference of the bottom. The cross-section of the sidewall, hence the cross-section of the suction chamber corresponds to the geometry of the bottom. If for instance the bottom is of circular symmetric geometry the sidewall typically comprises a sleeve-like or cylindrical geometry. Otherwise, if the bottom comprises a rectangular, triangular, pentagonal or hexagonal shape also the sidewall will be configured and designed accordingly.

The sidewall and the bottom typically form a cup-shaped receptacle being open towards an end located opposite to the bottom. This end, presently denoted as an upper end, is engageable, typically sealingly engageable with the flexible portion of the container's wall structure. In other words, the suction chamber is sealable by the flexible portion of the wall structure. In this way and when generating a low pressure level inside the suction chamber via the suction outlet the flexible portion is dragged or sucked into the suction chamber, typically towards the bottom of the body.

According to a further embodiment an upper end of the sidewall facing away from the bottom is gastight engageable with the flexible portion of the container's wall structure. Typically, the geometry and shape of the upper end of the sidewall of the body mates and corresponds to the shape and geometric structure of the flexible portion of the container's wall structure. Typically, the flexible portion is substantially even-shaped or outwardly bulged so as to form a gastight seat with the upper end of the body's sidewall. In order to obtain a good and sufficiently gastight engagement of the sidewall and the flexible portion the body's sidewall is typically of sleeve-like shape. It may be of circular or oval geometry or cross-section.

According to a further embodiment the upper end of the sidewall of the body comprises a sealing gasket to abut in a gastight way with the container's flexible portion. The sealing gasket as well as the flexible portion of the container may comprise or may consist of an elastomeric material, such like natural or synthetic rubber exhibiting a mechanical flexibility and elasticity that provides good and sufficient gastight seal.

According to another embodiment a proximal end of the piercing element is located axially offset and recessed from the sidewall's upper end. The axial direction may coincide with the longitudinal direction of the piercing element. By having the proximal end of the piercing element axially offset from the sidewall's upper end, the proximal end of the piercing element, which is tipped or pointed is located inside the suction chamber and does not protrude axially therefrom. Having the tipped proximal end of the piercing element in a recessed position with regard to the axial dimension or axial extension of the body's sidewall a danger of inadvertent stitching can be reduced. In this way, patient safety can be improved.

According to a further embodiment the suction outlet is located in one of the bottom and the sidewall. Moreover, the suction outlet is connectable to a low pressure source. The low pressure source may comprise a closed container having a pressure level in its interior that is substantially lower than the environmental pressure or being substantially lower than the pressure inside the container filled with the liquid medicament. The low pressure source may be alternatively provided by means of a pump, typically by means of a suction pump that is operable to generate a pressure level inside the suction chamber that is lower than the pressure level inside the container.

The suction outlet may be directly connected to the low pressure source. Typically, there is provided a kind of a switch or regulator between the low pressure source and the suction outlet, so that generation of a rather low pressure level inside the suction chamber is controllable, e.g. by means of the regulator. In an embodiment the low pressure source is connectable to the suction outlet or is engageable with the suction outlet in a fluid transferring way only when the suction chamber sealingly engages with the flexible portion of the container's wall structure. In this way, lowering of the pressure inside the suction chamber requires a gastight connection of the suction chamber with the flexible portion of the container's wall structure.

According to another embodiment the connector is actually connected to a container filled with a liquid medicament and having a wall structure with at least one flexible portion. The suction chamber is typically sealed by the wall structure of the container. At least a section of the flexible portion of the wall structure confines the inner volume of the suction chamber or seals the suction chamber. The wall structure may be substantially rigid or flexible in its entirety. If the wall structure is rather rigid and inelastic it comprises at least an aperture or through opening that is sealed or covered by the flexible portion.

The mutual connection of the connector and the container is such, that at least a portion of the flexible portion of the container's wall structure is flexible, suckable or deformable into the interior of the suction chamber. It is for instance possible, that a rather rigid wall structure of the container is in gastight engagement with the sidewall of the body of the connector. Then, the flexible portion of the wall structure is entirely encircled or enclosed by the sidewall of the connector's body. In response to an application of a considerable low pressure via the suction outlet the flexible portion will bend and flex inwardly, hence towards the bottom of the body to get pierced by the proximal end of the piercing element.

It is also conceivable, that the flexible portion directly engages with the sidewall of the body, in particular with the upper end thereof. It is also conceivable, that the sidewall of the connector's body engages with the container in a region, in which the substantially rigid wall structure is connected to and with the flexible portion thereof. In any case and independent from the specific design and configuration of the container's wall structure and its flexible portion application of a low pressure level inside the suction chamber leads to a pressure induced deformation of the flexible portion that enables and governs a piercing of the flexible portion by the piercing element, thereby providing and gaining access to the interior of the container. As soon as the pressure inside the suction chamber raises or returns to an initial value the flexible portion returns into its initial configuration or state due to its inherent elasticity.

Accordingly and following a further embodiment the flexible portion of the wall structure of the container is deformable into the suction chamber in response to a pressure inside the suction chamber being lower than a pressure inside the container. The pressure inside the container may be substantially equal to the ambient pressure while the pressure in the suction chamber may be actively lowered in comparison thereto. A deformation of the flexible portion deeper into the suction chamber is typically accompanied by the flexible portion flexing and reaching towards the bottom of the connector's body from which the proximal end of the piercing element extends e.g. upwardly.

In a further embodiment the flexible portion is retractable or displaceable into the suction chamber and towards the bottom to get pierced by the piercing element, in particular by a proximally directed and tipped portion of the piercing element. The piercing element is typically fixed to the bottom and hence to the body of the connector. A repeated piercing and disconnection of the piercing element and the container is achievable by lowering and raising the pressure inside the suction chamber, respectively.

According to a further embodiment the flexible portion of the wall structure of the container comprises an elastomeric sealing material. Typically, the pierceable seal may be implemented like a septum known from pierceable cartridges of injection devices of pen-injector type. The piercable seal may comprise or consist of a natural or synthetic rubber, such like bromobutyl-rubber. The flexible portion comprises a geometric shape and thickness that enables a sufficient displacement or elastic deformation thereof into the suction chamber while simultaneously providing a gastight seal.

By making use of an elastomeric sealing material the flexible portion is repeatedly pierceable by a tipped proximal end of the piercing element. Even after a detachment of the flexible portion and the piercing element the elastomeric sealing material even seals the pierced section. It is so to say of self-healing type and provides repeated puncturing or penetration of a tipped piercing element without a substantial or measureable deterioration of its sealing capabilities.

According to a further embodiment the flexible portion of the container's sidewall relaxes into an initial non-pierced state when the pressure inside the suction chamber approaches the pressure inside the container. Thanks to the elastic properties of the material the flexible portion is made of, the flexible portion returns into its initial state once the pressure difference between the interior of the container and the suction chamber drops below a predefined minimum threshold. In this way a fluid transferring coupling and decoupling of the piercing element, hence of the connector and the container can be obtained only by regulating and modifying the pressure inside the suction chamber of the connector.

According to a further embodiment the connector further comprises a low-pressure reservoir or a suction pump that is connectable to the suction outlet. In this embodiment the low pressure reservoir and/or the suction pump or vacuum pump acts as a pressure source as mentioned above. By implementing or integrating the low pressure reservoir or a respective suction pump into the connector a comparatively high degree of system integration can be obtained. Consequently, the connector does not have to be manually connected to a low pressure source but is readily equipped with such a pressure source.

In another aspect the invention also relates to an injection device comprising a connector as described above.

In still another embodiment the injection device comprises also a container with a wall structure having at least a flexible portion that is in gastight connection with the suction chamber of the connector. Typically, the container is exchangeably connected to the connector and/or to the injection device. The injection device may comprise a compartment to accommodate the container. The injection device may be particularly configured for empty container replacement. Such a replacement procedure may be accompanied by operably disconnecting the connector and the container, by replacing an empty container with a new container filled with the medicament and by applying a comparatively low pressure to the suction chamber of the connector so as to establish a fluid transferring coupling of container and piercing element of the connector.

In another further embodiment, the container comprises a flexible bag comprising at least one of the following materials: thermoplastic elastomers (TPE), silicon rubber, butadiene rubber (BR), styrene butadiene rubber (SBR), styrene-ethylene/butylene-styrene type polymers (SEBS), LDPE, LLDPE, ethylene vinyl acetate (EVA), random copolymers of VP, polybutene-1, COC- or COP-based elastomers. The flexible bag may further comprise a comparatively thin layer of polymeric material. Then it may comprise or consist of one of the following materials or combinations thereof: MDPE, high-density polyethylene (HDPE), PP, in form of homopolymer, random or heterophasic copolymers, polybutene-1, COC, COP, polymethylene pentane, PET, Polyethylenterephthalat Glycol (PET-G), PBT, PC, SAN or MABS. In a further embodiment the container or the flexible bag comprises a transparent portion or is made of a transparent material to allow visual inspection of its content.

In the present context, the distal direction points in the direction of the dispensing end of the container, where the piercing element is connectable to the container. A proximal end or proximal direction denotes the end of the connector facing towards the container and its flexible side wall portion.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39); or

des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;
or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (~150kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or A, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

It will be further apparent to those skilled in the art that various modifications and variations can be made to the present invention without departing from the spirit and scope of the invention. Further, it is to be noted, that any reference numerals used in the appended claims are not to be construed as limiting the scope of the invention.

### Brief description of the drawings

In the following, an embodiment of the connector in combination with a respective container is described in detail by making reference to the drawings, in which:
- Fig. 1: schematically shows a container coupled to a connector in an initial configuration,
- Fig. 2: shows the assembly of container and connector according to Fig. 1 in a deployed or activated configuration and
- Fig. 3: schematically shows an injection device provided with a connector according to Figs. 1 and 2.

### Detailed description

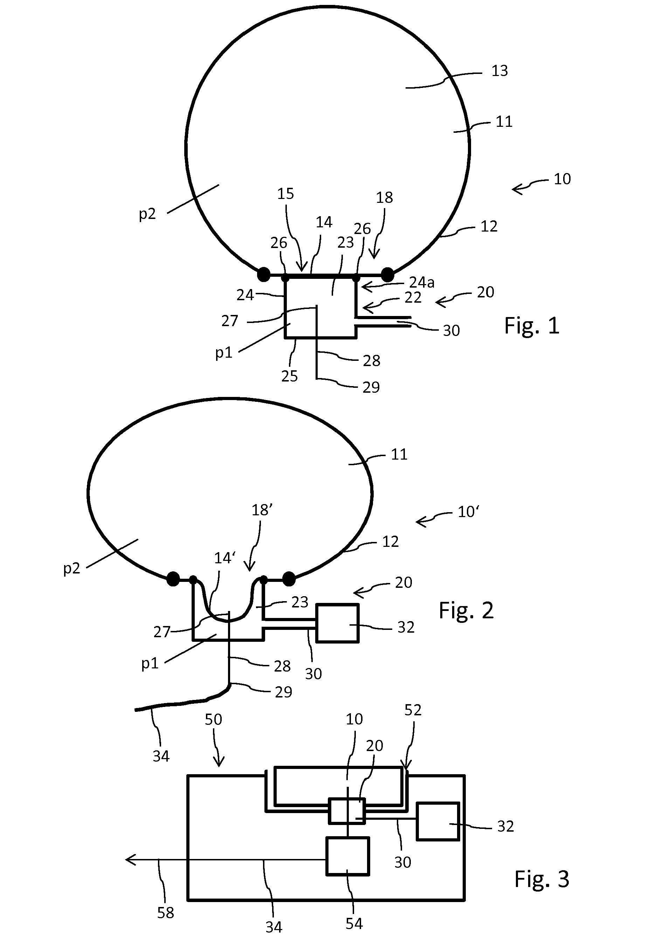

In Figs. 1 and 2 a connector 20 coupled or connected to a container 10 is illustrated in cross-section. The container 10 comprises a wall structure 12 confining an interior volume 11, in which a liquid medicament 13 is located. The container 10 may comprise a substantially rigid and inflexible wall structure 12 of arbitrary geometry. In one embodiment the wall structure 12 may be also flexible and elastically deformable. The container 10 further comprises a pierceable seal 14 that is located at one end section of the wall structure 12. Typically, the wall structure 12 comprises a through opening 15 or an aperture that is covered and closed by the pierceable seal 14. The pierceable seal 14 forms a flexible portion 18 of the wall structure 12.

There is further provided a connector 20 having a body 22 with a bottom 25 and a sidewall 24 attached thereto. The interior of the body 22 forms a suction chamber 23, which when attached to the container 10 is covered or closed by the flexible portion 18 of the wall structure 12 of the container 10. In the embodiment as shown in Figs. 1 and 2 the cylindrically or sleeve-like shaped sidewall 24 comprises a gasket 26 at an upper end section 24a facing away from the bottom 25. The flexible portion 18 extends all over the diameter or cross-section of the sidewall 24. Its extension is even larger than the cross-section of the sidewall 24. In other embodiments it is conceivable that the flexible portion 18 is smaller in diameter than the inner diameter or cross-section of the sidewall 24.

It is in fact only required, that at least a portion of the flexible portion 18 is located inside a volume or region confined by the sidewall 24 of the connector 20. The connector 20 further comprises a suction outlet 30 extending into the suction chamber 23. As shown in Figs. 1 and 2 the suction outlet 30 extends into and through the sidewall 24. It may be also located in the bottom 25 or may extend into the bottom 25. By means of the suction outlet 30, a low pressure level p1 is generatable inside the suction chamber 23 once it is effectively sealed by the wall structure 12 of the container 10.

Typically, the suction outlet 30 is connected to a low pressure source 32. The low pressure source 32 as shown in Fig. 2 may comprise an under or low pressure vessel. Furthermore, it may be implemented as a suction pump. By means of the low pressure source 32 a pressure level p1 inside the suction chamber 23 can be lowered to such a degree that it is substantially smaller than a pressure level p2 inside the container. As a consequence and as shown in Fig. 2, the flexible portion 18 of the wall structure 12 starts to extend into the suction chamber 23 as shown in Fig. 2. The degree of elastic deformation of the flexible portion 18 is controllable by modifying the pressure level p1 in comparison to the pressure level p2.

Furthermore the connector 20 comprises a piercing element 28 extending in an axial direction (z), which is aligned vertical in the illustration of Figs. 1 and 2. A proximal end 27 of the piercing element 28 extends into the suction chamber 23. Compared to the axial position of the upper end 24a of the sidewall 24, the proximal end 27 is in a recessed position. As seen in axial direction (z) the proximal end 27 does not protrude in proximal direction from a plane defined by the upper or proximal end 24a of the sidewall 24. The proximal end 27 is located distally from a proximal end of the connector 20 or its sidewall 24. In this way, the tipped and proximal end 27 of the piercing element 28 is protected by the circumferential sidewall 24. The sidewall 24 and the axially recessed location of the piercing element's proximal end 27 therefore act as a stitch protector. The distal end 29 of the piercing element 28 extends distally from the connector 20 or its body 22. It may be connected with a tubing 34 providing a transportation and flow of the liquid medicament 13 withdrawn from the interior 11 of the container 10.

As the pressure level p1 is lowered inside the suction chamber 23, the flexible portion 18' bulges outwardly or downwardly in Fig 2 and extends further into the suction chamber 23. In particular, with a lowering of the pressure level p1 the flexible portion 18' and hence the seal 14' extends towards the bottom 25 to such a degree that it gets pierced and intersected by the proximal end 27 of the piercing element 28. The hollow piercing element 28 then gets access to the interior 11 of the container 10 and hence to the liquid medicament 13 located therein.

Via a tubing 34 the liquid medicament 13 can be withdrawn from the container 10. By raising the pressure level p1 inside the suction chamber 23 to a level substantially equal to the pressure level p2 inside the container 10 the flexible portion 18 relaxes into its initial state as shown in Fig. 1. Consequently, the bulged flexible portion 18' as shown in Fig. 2 returns and retracts from the piercing element 28 and from the suction chamber 23 until a fluid transferring communication between the piercing element 28 and the interior 11 of the container 10 is abrogated. Typically, the flexible portion 18 or the seal 14 formed by the flexible portion 18 is made of an elastomeric material. The material properties as well as the geometry of the flexible portion 18 typically provide a self-healing functionality. When a tipped piercing element 28 is withdrawn from the flexible portion 18 the previously pierced section of the flexible portion reseals.

In Fig. 3 an injection device 50 cooperating with the assembly of container 10 and connector 20 is shown schematically. The injection device 50 comprises a compartment 52 to accommodate and to receive the container 10. The connector 20 is typically located inside the compartment 52 or at least adjoins the compartment 52 with its upper end 24a of its sidewall 24. The injection device 50 typically comprises a tubing 34 in fluid communication or fluidic connection with a distal end 29 of the piercing element 28.

Moreover, for a controlled feeding or withdrawal of the liquid medicament 13 from the container 10 the injection device 50 comprises a pump 54 by way of which the liquid medicament 13 can be withdrawn from the container 10 and by way of which it can be further provided to a device outlet 58 so that the liquid medicament 13 can be administered to a patient. In addition, the injection device 50 comprises a low pressure source 32 that is connected to the suction chamber 23 confined by the sidewall 24, the bottom 25 and the arrangement of flexible portion 18 and/or wall structure 12 of the container. By means of the low pressure source 32 typically in combination with some further control device or throttle the pressure level p1 inside the suction chamber 23 can be controlled and regulated. Lowering the pressure level p1 leads to reversible elastic deformation of the container's flexible portion 18, thereby piercing the seal 14 thereof to obtain access to the interior 11 of the container 10. In this way, controllable, repeated and reversible access to the liquid medicament 13 can be provided without the necessity of implementing a connector 20 with mechanically moving parts.

### List of reference numbers

- 10: container
- 11: interior
- 12: wall structure
- 13: liquid medicament
- 14: seal
- 15: through opening
- 18: flexible portion
- 20: connector
- 22: body
- 23: suction chamber
- 24: sidewall
- 24a: upper end
- 25: bottom
- 26: gasket
- 27: proximal end
- 28: piercing element
- 29: distal end
- 30: suction outlet
- 32: low pressure source
- 34: tubing
- 50: injection device
- 52: compartment
- 54: pump
- 58: device outlet

## Claims

1. A connector for a container (10) filled with a liquid medicament (13) and having a wall structure (12) with at least one flexible portion (18), the connector comprising:
- a body (22) forming a suction chamber (23) to engage with the flexible portion (18),
- a piercing element (28) extending through the body (22) and into the suction chamber (23),
- a suction outlet (30) in fluid communication with the suction chamber (23).

2. The connector according to claim 1, wherein the suction chamber (23) is formed by a bottom (25) and by a sidewall (24) connected thereto.

3. The connector according to claim 2, wherein an upper end (24a) of the sidewall (24) is gastight engageable with the flexible portion (18) of the container's (10) wall structure (12).

4. A connector according to claim 3, wherein the upper end (24a) of the sidewall (24) comprises a sealing gasket (26) to abut gastight with at least one of the container's wall structure (12) or the container's flexible portion (18).

5. The connector according to any one of the preceding claims 2-4, wherein a proximal end (27) of the piercing element (28) is located axially offset and recessed from the sidewall's (24) upper end (24a).

6. The connector according to any one of the preceding claims, wherein the suction outlet (30) is located in one of the bottom (25) and the sidewall (24) and wherein the suction outlet (30) is connectable or is connected to a low pressure source (32).

7. The connector according to any one of the preceding claims connected to the container (10) filled with the liquid medicament (13) and having a wall structure (12) with at least one flexible portion (18).

8. The connector according to claim 7, wherein the flexible portion (18) covers and/or seals the suction chamber 23.

9. The connector according to any one of the preceding claims 7 or 8 wherein the flexible portion (18) is deformable or stretchable into the suction chamber (23) in response to a pressure (p1) inside the suction chamber (23) lower than a pressure (p2) inside the container (10).

10. The container according to any one of the preceding claims 7 to 9, wherein the flexible portion (18) is displaceable into the suction chamber (23) and towards the bottom (25) to get pierced by the piercing element (28).

11. The connector according to any one of the preceding claims 7 to 10 wherein the flexible portion (18) comprises an elastomeric sealing material.

12. The connector according to any one of the preceding claims 7 to 11, wherein the flexible portion (18) relaxes into an initial non-pierced state, when the pressure difference (p2 - p1) between the interior (11) of the container (10) and the suction chamber (23) is below a predetermined threshold.

13. The connector according to any one of the preceding claims 6 to 12, wherein the low pressure source (32) comprises a low pressure reservoir or a suction pump connectable or connected to the suction outlet (30).

14. An injection device for administering a liquid medicament (13) and comprising a connector (20) according to any one of the preceding claims.

15. The injection device according to claim 14 further comprising a container 10 with a wall structure (12) having a flexible portion (18) being gastight in connection with the suction chamber (23) of the connector (20).
